# EUROPEAN PATENT APPLICATION

(11) **EP 4 257 146 A1**
(43) Date of publication of application: **11.10.2023**
(21) Application number: 21900451.2
(22) Date of filing: 24.11.2021
(51) Int. Cl.: A61K 45/00, A61K 31/517, A61K 31/7088, A61K 31/7105, A61K 31/713, A61K 39/395, A61P 35/00, A61P 35/02, A61P 43/00, C07K 16/18, C07K 16/22, C07K 16/28, C12N 15/113, C12N 15/115, C12N 15/12, C12Q 1/68, G01N 33/53, G01N 33/574

(54) **CYSTIC LYMPHANGIOMA TREATMENT DRUG**

(30) Priority: 04.12.2020 JP 2020201580; 13.04.2021 JP 2021067500
(71) Applicant: National University Corporation University Of Toyama, Toyama-shi, Toyama 930-8555 (JP)
(72) Inventor: YAMAMOTO, Seiji, Toyama-shi, Toyama 930-0194 (JP); SASAHARA, Masakiyo, Toyama-shi, Toyama 930-0194 (JP); HAMASHIMA, Takeru, Toyama-shi, Toyama 930-0194 (JP); OKUNO, Noriko, Toyama-shi, Toyama 930-0194 (JP)
(74) Representative: Müller-Boré & Partner Patentanwälte PartG mbB
(86) International application number: PCT/JP2021/042867
(87) International publication number: WO 2022/118696

(57) **Abstract**

A pharmaceutical composition for preventing or treating cystic lymphangioma comprising an agent that causes suppression of expression of amphiregulin, suppression of secretion of amphiregulin, and/or inhibition of binding of amphiregulin with an amphiregulin receptor, or an agent that causes suppression of expression of an amphiregulin receptor, suppression of activation of an amphiregulin receptor, and/or inhibition of binding of an amphiregulin receptor with amphiregulin, as an active ingredient.

## Description

### Technical Field

The present invention relates to a medicinal drug for cystic lymphangioma. More particularly, the present invention relates to a pharmaceutical composition and a kit for preventing or treating cystic lymphangioma, a method for testing cystic lymphangioma, a test kit for cystic lymphangioma, and a test agent for cystic lymphangioma.

### Background Art

Cystic lymphangioma is characterized by incidence of giant cysts. It has good outcomes from surgical resection. However, there are some cases in which cysts are unresectable, with the resection depending on the site of occurrence, such as a site adjacent to a child's respiratory tract and a site near an eye, nose, or mouth, and also in some cases, the treatment effect of sclerotherapy is insufficient. The development of a therapeutic agent for cystic lymphangioma based on molecular mechanisms has been desired.

PTL 1 discloses ligands for a protein associated with regulation of obesity, diabetes, and metabolic energy levels in animals, including humans. However, hundreds of diseases, including Alzheimer's disease and diabetes, are listed, and cystic lymphangioma is merely one of them. In addition, no substance associated with prevention or treatment of cystic lymphangioma is described.

### Citation List

### Patent Literature

PTL 1
Japanese Patent Publication No. 2007-523599

### Summary of Invention

### Technical Problem

A problem to be solved by the present invention is to provide a pharmaceutical composition and a kit for preventing or treating cystic lymphangioma comprising an agent that had not been reported as an active ingredient, a method for testing cystic lymphangioma, a test kit for cystic lymphangioma, and a test agent for cystic lymphangioma.

### Solution to Problem

The present invention covers the following embodiments.

Item 1. A pharmaceutical composition for preventing or treating cystic lymphangioma comprising an agent that causes suppression of expression of amphiregulin, suppression of secretion of amphiregulin, and/or inhibition of binding of amphiregulin with an amphiregulin receptor, as an active agent.

Item 2. The pharmaceutical composition according to Item 1, wherein the agent is selected from the group consisting of a neutralizing antibody against amphiregulin, an antigen binding fragment of a neutralizing antibody against amphiregulin, an antisense oligonucleotide against amphiregulin, a small interfering RNA against amphiregulin, a ribozyme against amphiregulin, and an aptamer against amphiregulin.

Item 3. A pharmaceutical composition for preventing or treating cystic lymphangioma comprising an agent that causes suppression of expression of an amphiregulin receptor, suppression of activation of an amphiregulin receptor, and/or inhibition of binding of an amphiregulin receptor with amphiregulin, as an active ingredient.

Item 4. The pharmaceutical composition according to Item 3, wherein the agent is selected from the group consisting of a neutralizing antibody against an amphiregulin receptor, an antigen binding fragment of a neutralizing antibody against an amphiregulin receptor, an antisense oligonucleotide against an amphiregulin receptor, a small interfering RNA against an amphiregulin receptor, a ribozyme against an amphiregulin receptor, an aptamer against an amphiregulin receptor, and a low-molecular compound that inhibits proliferation of lymphatic endothelial cells via an amphiregulin receptor.

Item 5. Use of an agent of either (a) or (b) for manufacturing a medicament for preventing or treating cystic lymphangioma:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

Item 6. A medicament for inhibiting proliferation of lymphatic endothelial cells comprising an agent of either (a) or (b) as an active ingredient:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

Item 7. A kit for preventing or treating cystic lymphangioma comprising an agent of either (a) or (b):
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

Item 8. A method for testing cystic lymphangioma comprising measuring amphiregulin or an amphiregulin receptor in a sample collected from a subject.

Item 9. A test kit for cystic lymphangioma comprising either (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.

Item 10. A test agent for cystic lymphangioma comprising either substance (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.

### Advantageous Effects of Invention

According to the invention, an agent that is effective for preventing and/or treating cystic lymphangioma, for which there has been no basic therapeutic agent, is provided.

### Brief Description of Drawings

Fig. 1

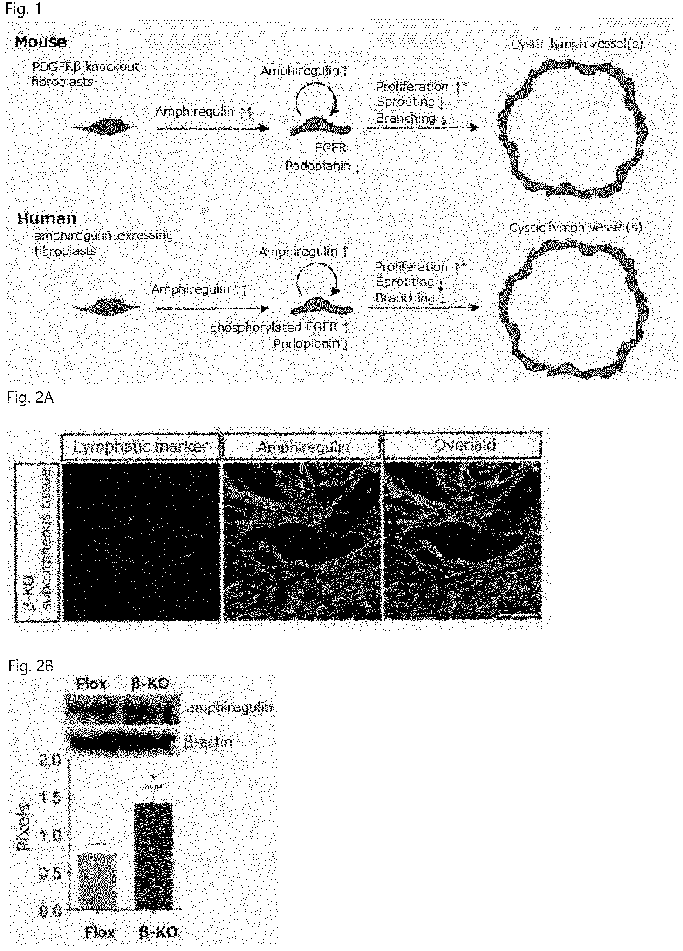

   Fig. 1 is a schematic view of molecular mechanisms of the formation of cystic abnormal lymph vessels in a mouse and human.
Fig. 2A
   Fig. 2A is immunohistological staining around lymphangioma of a PDGFRβ knockout mouse. Left: lymphatic marker (LYVE1); middle: amphiregulin; right: an overlaid image of lymphatic marker (LYVE1), amphiregulin, and nucleus. The scale bar is 50 µm.
Fig. 2B
   Fig. 2B is western-blot analysis of expression of amphiregulin in fibroblasts. Flox: control group; β-KO: a group of tamoxifen-administered PDGFRβ knockout mice. * is < 0.05.
Fig. 3A

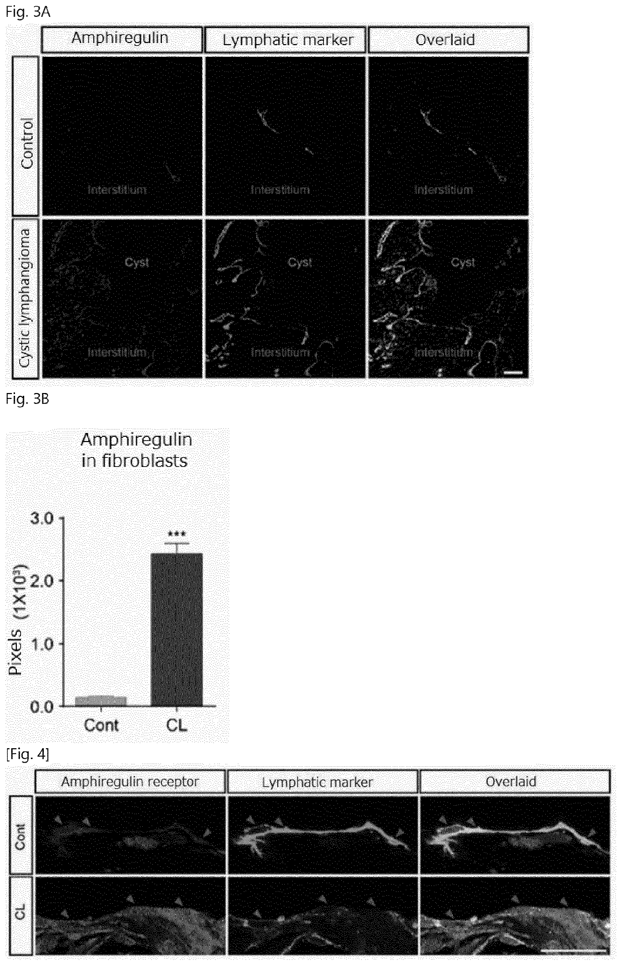

   Fig. 3A is immunohistological staining of a lesional tissue of lymph vessels derived from a patient with human cystic lymphangioma. Left: amphiregulin; middle: lymphatic marker (podoplanin); right: an overlaid image of amphiregulin, lymphatic marker (podoplanin), and nucleus. The scale bar is 100 µm.
Fig. 3B
   Fig. 3B is pixel analysis of expression of amphiregulin in fibroblasts. Cont: control group; CL: a group of patients with human cystic lymphangioma. *** is <0.001.
Fig. 4
   Fig. 4 is immunohistological staining of cystic lymphatic endothelial cells in a tissue derived from a patient with human cystic lymphangioma. Left: phosphorylated amphiregulin receptor (pEGFR); middle: lymphatic marker (podoplanin); right: an overlaid image of phosphorylated amphiregulin receptor (pEGFR), lymphatic marker (podoplanin), and nucleus. Cont: control group; CL: a group of patients with human cystic lymphangioma. The scale bar is 20 µm.
Fig. 5A

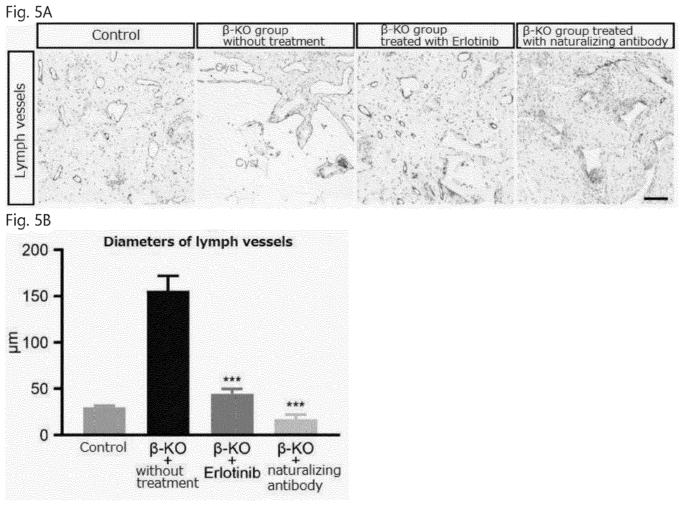

   Fig. 5A is morphology of LYVE1-positive lymph vessels. Left: a control group in which PDGFRβ is not knocked out; left middle: a group of PDGFRβ knockout mice without treatment; right middle: PDGFRβ knockout mice treated with Erlotinib; right: PDGFR β knockout mice treated with an amphiregulin-neutralizing antibody. The scale bar is 100 µm.
Fig. 5B
   Fig. 5B is results of statistical analysis of diameters of lymph vessels. *** is <0.001.

### Description of Embodiments

As used herein, the term "a subject" refers to a mammal, including a human, mouse, rat, bovine, horse, pig, dog, cat, rabbit, goat, and sheep. Preferably, the subject is a human or mouse. More preferably, the subject is a human.

As used herein, the term "treatment" refers to a cure or improvement of a disease or symptom, or suppression of a symptom. The term "prevention" refers to prevention of onset of a disease or symptom.

To develop a new drug, an animal model that reflects the clinical condition of a human is required. For cystic lymphangioma, mice having dilation of lymph vessels have been known to some extent. However, most of them are inappropriate as an animal model of human lymphangioma because anastomosis between a blood vessel and lymph vessel, such as dysfunction of blood platelets, occurs in the course of development and secondarily causes dilation of lymph vessels.

Recently, we developed a model of lymphangiogenesis in adult mice. By applying this model to uniquely produced PDGFRβ-conditioned systemic knockout mice (denoted as "PDGFRβ knockout mice" hereinafter) (Stem Cells, 2016 Mar; 34(3): 685-98. doi: 10.1002/stem.2212; EBioMedicine, 2018 May; 31: 190-201. doi: 10.1016/j.ebiom.2018.04.021.), cystic lymphangioma-like clinical conditions were successfully regenerated.

After searching for a factor of dilation of lymphatic vessels using the above mice, the inventors surprisingly found that amphiregulin, which had so far not attracted attention, was highly expressed in fibroblast cells around a lesional site. The invention was thus achieved.

According to a first aspect of the invention, provided is a pharmaceutical composition for preventing or treating cystic lymphangioma comprising an agent that causes suppression of expression of amphiregulin, suppression of secretion of amphiregulin, and/or inhibition of binding of amphiregulin with an amphiregulin receptor, as an active agent.

Secretion of amphiregulin indicates secretion of amphiregulin in secretory form to outside the cells (e.g., release from a cell membrane in a shedding manner).

Amphiregulin (AREG) is a glycoprotein that is a member of the epidermal growth factors (EGF) and has an action to promote growth of epidermal cells by interacting with an EGF receptor. Amphiregulin includes membrane-bound amphiregulin (also referred to as "amphiregulin proprotein" or "amphiregulin preproprotein") and secretory amphiregulin (also referred to as "amphiregulin protein"). For these amphiregulins, see Semin, Cell Dev Biol 2014 Apr; 28: 31-41. doi: 10.1016/j.semcdb.2014.01.005.

In one embodiment, amphiregulin is a protein having an amino acid sequence that is at least 90% identical to the amino acid sequence represented by SEQ ID NO. 1, 2, 3, 4, 5, or 6.

SEQ ID NO. 1 is an amino acid sequence of human membrane-bound amphiregulin protein consisting of 252 amino acids (GenBank Accession Number: NP_001648.1). SEQ ID NO. 2 is human secretory amphiregulin protein and corresponds to a portion of the 101^{th} to 187^{th} amino acids in the amino acid sequence of human membrane-bound amphiregulin protein.

SEQ ID NO. 3 is an amino acid sequence of mouse membrane-bound amphiregulin protein consisting of 248 amino acids (GenBank Accession Number: NP_033834.1). SEQ ID NO. 4 is mouse secretory amphiregulin protein and corresponds to a portion of the 100^{th} to 180^{th} amino acids in the amino acid sequence of mouse membrane-bound amphiregulin protein.

SEQ ID NO. 5 is an amino acid sequence of rat membrane-bound amphiregulin protein consisting of 243 amino acids (GenBank Accession Number: NP 058819.1). SEQ ID NO. 6 is rat secretory amphiregulin protein and corresponds to a portion of 97^{th} to 178^{th} amino acids in the amino acid sequence of rat membrane-bound amphiregulin protein.

In more preferred embodiments, amphiregulin is a protein having an amino acid sequence that is at least 95% identical, at least 98% identical, at least 99% identical, or 100% identical to the amino acid sequence represented by SEQ ID NO. 1, 2, 3, 4, 5, or 6.

In more preferred embodiments, amphiregulin is a protein having an amino acid sequence that is at least 90% identical, at least 95% identical, at least 98% identical, at least 99% identical, or 100% identical to the amino acid sequence represented by SEQ ID NO. 1 or SEQ ID NO. 2, and having an action to promote proliferation of cells that express EGFR and HER2, or cells that express EGFR but do not express HER2.

In more preferred embodiments, amphiregulin is a protein having an amino acid sequence that is at least 90% identical, at least 95% identical, at least 98% identical, at least 99% identical, or 100% identical to the amino acid sequence represented by SEQ ID NO. 3 or SEQ ID NO. 4, and having an action to promote proliferation of cells that express EGFR and HER2, or cells that express EGFR but do not express HER2.

Fig. 1 shows molecular mechanisms of the formation of cystic abnormal lymph vessels in a mouse and human. On the upper side of Fig. 1, the upward arrows indicate an increase in the expression amount of molecules in fibroblasts around lymph vessels of PDGFRβ knockout mouse fibroblasts or an increase in the expression of molecules or an increase in proliferation activity of lymphatic endothelial cells, compared with fibroblasts around lymph vessels of a normal mouse. The downward arrows indicate a decrease in the expression amount of molecules in lymphatic endothelial cells or a decrease in sprouting or branching of lymph vessels. On the lower side of Fig. 1, the upward arrows indicate an increase in the expression amount of molecules in fibroblasts around lymph vessels of patients with cystic lymphangioma or an increase in the expression of molecules or an increase in phosphorylated proteins, compared with fibroblasts around lymph vessels of a normal human. The downward arrows indicate a decrease in an expression amount of molecules in lymphatic endothelial cells or a decrease in sprouting or branching of lymph vessels.

Without wishing to be bound by any specific hypothesis or theory, according to the present studies of the inventors, it was revealed that amphiregulin was excessively secreted from fibroblast cells around lymph vessels in PDGFRβ knockout mice, which induces secretion of amphiregulin from lymphatic endothelial cells and excessive proliferation of lymphatic endothelial cells. It was also revealed that, also in human cystic lymphangioma, amphiregulin was excessively secreted from fibroblast cells around lymph vessels, which induces secretion of amphiregulin from lymphatic endothelial cells and excessive proliferation of lymphatic endothelial cells. This is the molecular mechanism of onset and exacerbation of cystic lymphangioma.

In some embodiments, the agent that causes suppression of expression of amphiregulin, suppression of secretion of amphiregulin, and/or inhibition of binding of amphiregulin with an amphiregulin receptor is selected from the group consisting of a neutralizing antibody against amphiregulin, an antigen binding fragment of a neutralizing antibody against amphiregulin, an antisense oligonucleotide against amphiregulin, a small interfering RNA against amphiregulin, a ribozyme against amphiregulin, and an aptamer against amphiregulin. Containing one or more such agents as an active ingredient is effective for the prevention or treatment of cystic lymphangioma.

Methods for producing an antibody, including a method for producing a neutralizing antibody, are well known. (For example, see Antibodies: A Laboratory Manual (1988) by Ed Harlow and David Lane, Cold Spring Harbor Laboratory Press, Cold Spring Harbor, New York. For antibodies, antibodies according to various embodiments of the invention may be a polyclonal antibody, a monoclonal antibody, a chimeric antibody, a single-stranded antibody, a Fab fragment, and a fragment that is produced with a Fab expression library.)

For the production of an antibody, various hosts, including goats, rabbits, rats, mice, and humans, may be immunized by injection with the polypeptide or any fragment or oligopeptide thereof that has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, keyhole limpet hemocyanin, and dinitrophenol.

A monoclonal antibody may be prepared using any technique that provides for the production of antibody molecules by continuous cell lines in culture. It includes, but is not limited to, the hybridoma technique, the human B-cell hybridoma technique, and the EBV-hybridoma technique (essentially as described in Kohler, G. et al. (1975), Nature 256: 495-497; Kozbor, D. et al. (1985), J. Immunol. Methods 81: 31-42; Cote, R. J. et al. (1983), Proc. Natl. Acad. Sci. 80: 2026-2030; Cole, S. P. et al. (1984), Mol. Cell Biol. 62: 109-120).

An antigen binding fragment of a neutralizing antibody means an antigen bonding portion of an antibody molecule and includes any polypeptide (for example, a molecule including one or more CDRs) including Fab, Fab', F(ab')₂, Fv, a single-stranded antibody (scFv), a chimeric antibody, a bispecific antibody (diabody), and at least a part of immunoglobulin that is sufficient to provide a specific binding property with an antigen. An antigen binding fragment of a neutralizing antibody can be produced by enzymatic digestion of the antibody molecule. For example, a Fab fragment may be produced by reducing a F(ab') ₂ fragment that is generated by pepsin digestion of an antibody molecule or disulfide bridges of the F(ab') ₂ fragment. Alternatively, Fab expression libraries may be constructed to allow rapid and easy identification of monoclonal Fab fragments with the desired specificity (for example as described in Huse, W. D. et al. (1989), Science 254: 1275-1281)_{∘}

An antisense oligonucleotide may function as an inhibitor of gene expression of amphiregulin. The antisense oligonucleotide includes an antisense RNA molecule and an antisense DNA molecule. It serves to directly inhibit the translation of amphiregulin mRNA by binding amphiregulin mRNA, thus preventing the translation of amphiregulin into a protein or increasing the degradation of amphiregulin mRNA and then decreasing the expression level (or expression amount) of amphiregulin. For example, an antisense oligonucleotide having at least 15 bases that is complementary to the non-repetitive region of the mRNA transcription sequence encoding amphiregulin may be synthesized by a conventional phosphodiester technique. Methods that are used in an antisense technique for specifically inhibiting gene expression of a gene having a known sequence are well known in the art (for example, see Stein et al. (1988), Nucl. Acids Res. 16). The antisense oligonucleotide may be prepared by known methods such as solid-phase phosphoramidite chemical synthesis. The antisense oligonucleotide may also be generated by transcribing a DNA sequence encoding an RNA molecule in vitro or in vivo. Such a DNA sequence may be incorporated into a wide variety of vectors incorporating an appropriate RNA polymerase promoter such as a T7 or SP6 polymerase promoter.

A small interfering RNA (siRNA) against amphiregulin may also function as an inhibitor of gene expression of amphiregulin. The gene expression of amphiregulin may be decreased by contacting siRNA that is a small double-stranded RNA (dsRNA) or a vector that produces the siRNA with a tissue or cells of a subject. As a result, RNA interference (RNAi) occurs, and gene expression of amphiregulin is specifically inhibited. Methods for selecting an appropriate dsRNA against a gene having a known sequence and an appropriate vector encoding the dsRNA are well known (for example, see Elbashir et al. (2001), Nature AIV. 494-498; Harborth et al. (2003), Antisense Nucleic Acid Drug Dev. 13: 83-106; and Semizarov et al. (2003), Proc Natl Acad Sd USA 100: 6347-635).

A ribozyme may also function as an inhibitor of gene expression of amphiregulin. The ribozyme is an enzymatic RNA molecule that is capable of catalyzing a specific cleavage of RNA. The action mechanism of the ribozyme includes cleavage of the nucleotide strand followed by sequence-specific hybridization of a complementary target RNA and the ribozyme molecule. A ribozyme molecule having a hairpin or hammerhead motif that specifically and efficiently catalyzes cleavage of a nucleotide strand of amphiregulin mRNA is useful in the scope of the invention. A specific ribozyme cleavage site in any potential RNA target can be identified by scanning a target molecule for the ribozyme cleavage sites including the sequences GUA, GUU, and GUC. Once identified, a short RNA having 15 to 20 ribonucleotides corresponding to the region of the target gene including one or more cleavage sites may be evaluated for the predicted structural characterization, such as secondary structure. The ribozyme may be prepared by known methods such as solid-phase phosphoramidite chemical synthesis.

An aptamer is an oligonucleotide that binds the target with high affinity and specificity. An aptamer is a class of molecule that is a replacement of an antibody in molecular recognition. The aptamer against amphiregulin is an oligonucleotide that has the ability to recognize amphiregulin with high affinity and specificity. As is well known in the art, an aptamer may be selected using the SELEX method (systematic evolution of ligands by exponential enrichment).

The agent according to the first aspect of the invention may cause a change in amphiregulin inside and/or outside the cells. For example, the agent may completely or partially inhibit the function of amphiregulin secreted outside the cells. Alternatively, the agent may inhibit the translation of amphiregulin by cleaving or degrading an RNA molecule encoding amphiregulin in cells.

The agents of various embodiments according the first aspect of the invention may control one or more of the following: expression, secretion, localization, and/or activity of a molecule involved in onset or development of cystic lymphangioma upstream or downstream from amphiregulin.

According to a second aspect of the invention, provided is a pharmaceutical composition for preventing or treating cystic lymphangioma comprising an agent that causes suppression of expression of an amphiregulin receptor, suppression of activation of an amphiregulin receptor, and/or inhibition of binding of an amphiregulin receptor with amphiregulin, as an active ingredient.

An amphiregulin receptor includes dimers that consist of two or more molecules of the ErbB family (EGF receptor family) and that receive amphiregulin. The ErbB family includes four receptor-type tyrosine kinases: EGFR (epidermal growth factor receptor, also referred to as "HER1" or "ErbB1"), HER2 (also referred to as "ErbB2"), HER3 (also referred to as "ErbB3"), and HER4 (also referred to as "ErbB4"). Activation of the receptor is caused by phosphorylation of tyrosine residue in the receptor.

In one embodiment, an amphiregulin receptor is a homodimer of proteins each having an amino acid sequence that is at least 90% identical to the amino acid sequence represented by SEQ ID NO. 7, 8, 9, 13, 14, 15, 16, or 17.

SEQ ID NO. 7 is an amino acid sequence of human epidermal growth factor receptor protein consisting of 1,210 amino acids (GenBank Accession Number: NP_005219.2).

SEQ ID NO. 8 is an amino acid sequence of mouse epidermal growth factor receptor protein consisting of 1,210 amino acids (GenBank Accession Number: NP_997538.1).

SEQ ID NO. 9 is an amino acid sequence of rat epidermal growth factor receptor protein consisting of 1,209 amino acids sequence (GenBank Accession Number: ADT91285.1).

SEQ ID NO. 13, 14, 15, 16, and 17 are isoforms of human epidermal growth factor protein of SEQ ID NO. 7 (GenBank Accession Number: NP_001333826.1, NP_001333827.1 NP_001333828.1, NP_001333829.1, NP_001333870.1, respectively).

In more preferred embodiments, an amphiregulin receptor is a homodimer of proteins each having an amino acid sequence that is at least 95% identical, at least 98% identical, at least 99% identical, or 100% identical to the amino acid sequence represented by SEQ ID NO. 7, 8, 9, 13, 14, 15, 16, or 17.

In more preferred embodiments, an amphiregulin receptor is a homodimer of proteins each having an amino acid sequence that is at least 90% identical, at least 95% identical, at least 98% identical, at least 99% identical, or 100% identical to the amino acid sequence represented by SEQ ID NO. 7.

In more preferred embodiments, an amphiregulin receptor is a homodimer of proteins each having an amino acid sequence that is at least 90% identical, at least 95% identical, at least 98% identical, at least 99% identical, or 100% identical to the amino acid sequence represented by SEQ ID NO. 8.

In another embodiment, an amphiregulin receptor is a heterodimer of a protein having an amino acid sequence that is at least 90% identical to the amino acid sequence represented by SEQ ID NO. 7, 13, 14, 15, 16, or 17, and a protein having an amino acid sequence that is at least 90% identical to the amino acid sequence represented by SEQ ID NO. 10, a heterodimer of a protein having an amino acid sequence that is at least 90% identical to the amino acid sequence represented by SEQ ID NO. 8 and a protein having an amino acid sequence that is at least 90% identical to the amino acid sequence represented by SEQ ID NO. 11, or a heterodimer of a protein having an amino acid sequence that is at least 90% identical to the amino acid sequence represented by SEQ ID NO. 9 and a protein having an amino acid sequence that is at least 90% identical to the amino acid sequence represented by SEQ ID NO. 12.

SEQ ID NO. 10 is an amino acid sequence of human HER2 protein consisting of 1,255 amino acids (GenBank Accession Number: NP_001005862.1).

SEQ ID NO. 11 is an amino acid sequence of mouse HER2 protein consisting of 1,256 amino acids (GenBank Accession Number: NP_001003817.1).

SEQ ID NO. 12 is an amino acid sequence of rat HER2 protein consisting of 1,259 amino acids (GenBank Accession Number: NP_058699.2).

In another embodiment, an amphiregulin receptor is a heterodimer of a protein having an amino acid sequence that is at least 95% identical, at least 98% identical, at least 99% identical, or 100% identical to the amino acid sequence represented by SEQ ID NO. 7, 13, 14, 15, 16, or 17, and a protein having an amino acid sequence that is at least 95% identical, at least 98% identical, at least 99% identical, or 100% identical to the amino acid sequence represented by SEQ ID NO. 10, a heterodimer of a protein having an amino acid sequence that is at least 95% identical, at least 98% identical, at least 99% identical, or 100% identical to the amino acid sequence represented by SEQ ID NO. 8, and a protein having an amino acid sequence that is at least 95% identical, at least 98% identical, at least 99% identical, or 100% identical to the amino acid sequence represented by SEQ ID NO. 11, or a heterodimer of a protein having an amino acid sequence that is at least 95% identical, at least 98% identical, at least 99% identical, or 100% identical to the amino acid sequence represented by SEQ ID NO. 9, and a protein having an amino acid sequence that is at least 95% identical, at least 98% identical, at least 99% identical, or 100% identical to the amino acid sequence represented by SEQ ID NO. 12.

In some embodiments, the agent that causes suppression of expression of an amphiregulin receptor, suppression of activation of an amphiregulin receptor, and/or inhibition of binding of an amphiregulin receptor with amphiregulin is selected from the group consisting of a neutralizing antibody against an amphiregulin receptor, an antigen binding fragment of a neutralizing antibody against an amphiregulin receptor, an antisense oligonucleotide against an amphiregulin receptor, a small interfering RNA against an amphiregulin receptor, a ribozyme against an amphiregulin receptor, an aptamer against an amphiregulin receptor, and a low-molecular compound that inhibits proliferation of lymphatic endothelial cells via an amphiregulin receptor. Containing one or more such agents as an active ingredient is effective for the prevention or treatment of cystic lymphangioma.

The neutralizing antibody, the antigen binding fragment of the neutralizing antibody, the antisense oligonucleotide, the small interfering RNA, the ribozyme, and the aptamer are as described in the first aspect of the invention. The neutralizing antibody against an amphiregulin receptor, the antigen binding fragment of the neutralizing antibody, the antisense oligonucleotide, the small interfering RNA, the ribozyme, and the aptamer may be produced by those skilled in the art based on a well-known technique in the art.

The low-molecular compound that inhibits proliferation of lymphatic endothelial cells via an amphiregulin receptor includes low-molecular compounds such as an EGFR tyrosine kinase inhibitor, an HER2 tyrosine kinase inhibitor, and a tyrosine kinase inhibitor of both EGFR and HER2. For example, the low-molecular compound includes Gefitinib, Erlotinib, Afatinib, Osimertinib, Dacomitinib, Lapatinib, Vandetanib, Peritinib, Canertinib, Neratinib, Nazartinib, Lazertinib, Icotinib, Rociletinib, Ormucinib, CK-101(N-(3-(2-((2,3-Difluoro-4-(4-(2-hydroxyethyl)piperazin-1-yl)phenyl)amino)quinazolin-8-yl)phenyl)acrylamide), BMS-690514((3R,4R)-4-amino-1-[[4-(3-methoxyanilino)pyrrolo[2,1-f][1,2,4]triazin-5-yl]methyl]piperidin-3-ol), AEE788(6-{4-[(4-Ethyl-1-piperazinyl)methyl]phenyl}-N-[(1R)-1-phenylethyl]-1H-pyrrolo[2,3-d]pyrimidin-4-amine, CID 10297043, NVP-AEE 788, and [6-[4-[(4-Ethylpiperazin-1-yl)methyl]phenyl]-7H-pyrrolo[2,3-d]pyrimidin-4-yl]-((R)-1-phenylethyl)amine).

The agents of various embodiments according to the second aspect of the invention may control one or more of the following: expression, secretion, localization, and/or activity of a molecule involved in onset or development of cystic lymphangioma upstream or downstream from amphiregulin.

Each of the pharmaceutical compositions of the first and second aspects of the invention is targeted to a mammal, including a human, as a subject, and is administered preferably to a human or mouse, more preferably to a human.

Although the agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, may each be used as a single agent, either of the agents may be used for the prevention or treatment of cystic lymphangioma in combination with interferon α, propranolol, an anticancer drug such as vincristine, bisphosphonate, and octreotide.

The combination with one or more of these drugs not only improves a prevention or treatment effect of cystic lymphangioma by the agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, but also may reduce the dose compared to the case in which a single drug is used. Thus, it is preferable in terms of reduction of side effects.

The pharmaceutical composition of each of the first and second aspects of the invention may be administered to a human in various forms. Such forms may be, for example, an oral preparation, an injectable preparation, an external preparation such as an ointment preparation, and an adhesive patch. These preparations may be produced by methods known to those skilled in the art. In preparation, commonly used pharmaceutically acceptable carriers such as a binder, a disintegrant, a lubricant, a colorant, a flavoring agent, a deodorant, a buffer, a stabilizer, a tonicity agent, a solvent, an excipient, a solubilizer, a dispersant, a suspending agent, a preservative a pain reliever, a pigment, and a perfume may be used.

Among oral preparations, for a solid preparation, the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, an excipient, and, if required, a carrier such as a binder, a disintegrant, a lubricant, a colorant, a flavoring, and a deodorant, is mixed and processed in a standard method to form tablets, coated tablets, granules, powders, capsules, or dry syrup. For a liquid oral preparation, the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, a carrier such as a flavoring agent, a buffer, a stabilizer, and a deodorant, is used and processed in a standard method to form internal fluid, syrup, or an elixir.

For an injectable preparation, the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, is mixed with a pH-adjusting agent, a buffer, a stabilizer, a tonicity agent, or a local anesthetic, and processed in a standard method to form a subcutaneous injectable preparation, an intramuscular injectable preparation, or an intravenous injectable preparation.

Among external preparations, an ointment such as a paste, a cream, and a gel may be prepared by mixing a base including the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin with a stabilizer, a wetting agent, or a preservative as required, and treating in a standard method. The base includes, for example, white petrolatum, paraffin, glycerin, cellulose derivatives, polyethylene glycol, silicone, and bentonite. The preservative includes methyl p-hydroxybenzoate, ethyl p-hydroxybenzoate, and propyl p-hydroxybenzoate.

Among external preparations, an adhesive patch may be produced by applying an ointment, a cream, a gel, or a paste to a common support by a standard method. The support is preferably a woven cloth of cotton, staple fibers, or chemical fibers, a non-woven cloth, a film of soft vinyl chloride, polyethylene, or polyurethane, or a foam sheet.

The drug that can be used in combination may be an administration form similar to that of the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin. When the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin and another drug, is used in combination, they may be a single administration form including them, or two or more administration forms each of which includes either of them. As long as the object of the invention is achieved, separately prepared preparations may be administered to the same subject simultaneously or at different times.

The amount of the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, to be contained in each of the preparations may vary depending on the condition, body weight, age, and sex of a patient to which the agent is administered, or other condition, and may be determined appropriately depending on the dosage form. In general, the dose per day and per 1 kg of body weight of a subject may be as follows: in the case of an oral preparation, from about 1 ng to 1 g; in some embodiments, from 1 µg to 1 g; in some embodiments, from 0.1 mg to 100 mg; in the case of an injectable preparation, from about 1 ng to 500 mg; in some embodiments, from 1 µg to 500 mg; in some embodiments, from 0.1 mg to 50 mg; in the case of a suppository or an external preparation, from about 1 ng to 1g; in some embodiments, 1 µg to 1 g; in some embodiments, 0.1 mg to 100 mg. The preparation may be administered once in a day or may be divided into twice to four times a day.

The amount of the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, to be contained in the medicament may be determined appropriately depending on the dosage form. Per dosage unit of an adult, the amount may be as follows: in the case of an oral preparation, from about 10 ng to 10 g; in some embodiments, from 10 µg to 1 g; in some embodiments, from 1 mg to 1 g; in the case of an injectable preparation, from about 10 ng to 5 g; in some embodiments, from 10 µg to 5 g; in some embodiments, from 1 mg to 500 mg; in the case of a suppository or an external preparation, from about 10 ng to 10 g; in some embodiments, from 10 µg to 1 g; in some embodiments, from 1 mg to 1 g. The preparation may be administered once in a day or may be divided into twice to four times a day.

In some embodiments, when the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, is an antibody, an antigen binding fragment of an antibody, or an aptamer, the dose of the agent per day and per 1 kg of body weight of a subject is in the range of from 1 ng to 1 g; in some embodiments, 1 µg/day/kg to about 1 g/day/kg; in some embodiments, about 1 µg/day/kg to about 100 mg/day/kg; and in some embodiments, about 1 µg/day/kg to about 10 mg/day/kg.

In some embodiments, when the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, is an antisense oligonucleotide, a small interfering RNA, a ribozyme, or a low-molecular compound, the dose of the agent per day and per 1 kg of body weight of a subject is in the range of from 1 ng to 1 g; in some embodiments, from 1 µg/day/kg to about 1 g/day/kg; in some embodiment, from about 1 µg/day/kg to about 100 mg/day/kg; and in some embodiments, from about 1 µg/day/kg to about 10 mg/day/kg.

According to a third aspect of the invention, provided is use of an agent of either (a) or (b) for manufacturing a medicament for preventing or treating cystic lymphangioma:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

The agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, are described with respect to a pharmaceutical composition of the first aspect of the invention and a pharmaceutical composition of the second aspect of the invention, respectively.

According to a fourth aspect of the invention, provided is a medicament for inhibiting proliferation of lymphatic endothelial cells comprising an agent of either (a) or (b) as an active ingredient:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

The agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, are described with respect to a pharmaceutical composition of the first aspect of the invention and a pharmaceutical composition of the second aspect of the invention, respectively.

Although the agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, may each be used as a single agent, either of the agents may be used for inhibiting proliferation of lymphatic endothelial cells in combination with interferon α, propranolol, an anticancer drug such as vincristine, bisphosphonate, and octreotide.

The medicament according to the fourth aspect of the invention may be administered to a human in various forms. Such forms may be, for example, an oral preparation, an injectable preparation, an external preparation such as an ointment preparation, and an adhesive patch. These preparations may be produced by methods known to those skilled in the art. In preparation, commonly used pharmaceutically acceptable carriers such as a binder, a disintegrant, a lubricant, a colorant, a flavoring agent, a deodorant, a buffer, a stabilizer, a tonicity agent, a solvent, an excipient, a solubilizer, a dispersant, a suspending agent, a preserva tive, a pain reliever, a pigment, and a perfume may be used.

The lymphatic endothelial cells are those derived from a subject that is a mammal. The subject is preferably a human or mouse, more preferably a human.

The amount of the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin to be contained in a medicament, may be determined depending on the condition of a patient to which the agent is administered or on the dosage form. In general, per dosage unit of an adult, the amount may be as follows: in the case of an oral preparation, from about 10 ng to 10 g; in some embodiments, from 10 µg to 1 g; in some embodiments, from 1 mg to 1 g; in the case of an injectable preparation, from about 10 ng to 5 g; in some embodiments, from 10 µg to 5 g; in some embodiments, from 1 mg to 500 mg; in the case of a suppository or an external preparation, from about 10 ng to 10 g; in some embodiments, from 10 µg to 1 g; in some embodiments, from 1 mg to 1 g. The preparation may be administered once in a day or may be divided into twice to four times a day.

The amount of the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin, to be contained in each of the preparations per day may vary depending on the condition, body weight, age, and sex of a patient to which the agent is administered, or other condition. In general, per dosage unit of an adult, the amount may be as follows: in the case of an oral preparation, from about 10 ng to 10 g; in some embodiments, from 10 µg to 1 g; in some embodiments, from 1 mg to 1 g; in the case of an injectable preparation, from about 10 ng to 5 g; in some embodiments, from 10 µg to 5 g; in some embodiments, from 1 mg to 500 mg; in the case of a suppository or an external preparation, from about 10 ng to 10 g; in some embodiments, from 10 µg to 1 g; in some embodiments, from 1 mg to 1 g. The preparation may be administered once in a day or may be divided into twice to four times a day.

According to a fifth aspect of the invention, provided is a kit preventing or treating cystic lymphangioma comprising an agent of either (a) or (b):
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

The agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, are described with respect to a pharmaceutical composition of the first aspect of the invention and a pharmaceutical composition of the second aspect of the invention, respectively.

The kit may further comprise instructions for conducting a test. The kit may further comprise a container for containing the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin. The kit may further comprise a solvent for dissolving or suspending the agent that causes suppression of expression of amphiregulin, suppression of secretion of amphiregulin, and/or inhibition of binding of amphiregulin with an amphiregulin receptor, or the agent that causes suppression of expression of an amphiregulin receptor, suppression of activation of an amphiregulin receptor, and/or inhibition of binding of an amphiregulin receptor with amphiregulin. The kit may further comprise a preservative or a pH control agent.

According to a sixth aspect of the invention, a method for testing cystic lymphangioma comprising measuring amphiregulin or an amphiregulin receptor in a sample collected from a subject is provided.

The subject is a mammal, including, preferably, a human or mouse, more preferably a human.

The sample collected from a subject may be a tissue or cells. For example, the sample includes a lymphatic tissue, lymphatic endothelial cells, and fibroblast cells around one or more lymph vessels.

As used herein, the "test" of cystic lymphangioma includes determination of cystic lymphangioma, determination of cystic lymphangioma (a responder) for which a therapeutic agent is effective (companion diagnosis), determination of a prevention effect of cystic lymphangioma, determination of a treatment effect of cystic lymphangioma, a method for testing for aiding diagnosis (particularly, early diagnosis) of cystic lymphangioma, and a method for testing for aiding treatment (particularly, early treatment) of cystic lymphangioma. The "determination" of cystic lymphangioma not only includes determination of presence or absence of cystic lymphangioma but also preventive determination of the possibility of incidence of cystic lymphangioma, prediction of prognosis of cystic lymphangioma after the treatment, and determination of the treatment effect of a therapeutic agent for cystic lymphangioma.

Amphiregulin or an amphiregulin receptor is found in a sample collected from a subject. The measurement values of these proteins correlate with cystic lymphangioma. Specifically, the expression of amphiregulin and an amphiregulin receptor in a sample is significantly higher in a subject suffering from cystic lymphangioma than in a normal subject. In addition, the expression of amphiregulin and an amphiregulin receptor in a sample is significantly higher in a PDGFRβ-knockout mouse than in a normal mouse.

Thus, when the level of amphiregulin in a sample collected from a subject is the same as or lower than the level of amphiregulin in a sample collected from a normal subject, it means that the possibility of incidence of cystic lymphangioma is low in the subject and can be so determined. Hereinafter, "level" refers to concentration, amount, or signal strength. When the level of amphiregulin in a sample collected from a subject is the same as or higher than the average value or the middle value of the level of amphiregulin in samples collected from patients with cystic lymphangioma or a cutoff value that distinguishes between the level of amphiregulin in samples collected from patients with cystic lymphangioma and the level of amphiregulin in samples collected from normal subjects, it means that the possibility of incidence of cystic lymphangioma is high in the subject and can be so determined.

Similarly, when the level of an amphiregulin receptor in a sample collected from a subject is the same as or lower than the level of an amphiregulin receptor in a sample collected from a normal subject, it means that the possibility of incidence of cystic lymphangioma is low in the subject and can be so determined. When the level of an amphiregulin receptor in a sample collected from a subject is the same as or higher than the average value or the middle value of the level of an amphiregulin receptor in samples collected from patients with cystic lymphangioma or a cutoff value that distinguishes between the level of an amphiregulin receptor in samples collected from patients with cystic lymphangioma and the level of an amphiregulin receptor in samples collected from normal subjects, it means that the possibility of incidence of cystic lymphangioma is high in the subject and can be so determined.

Further, a method for testing cystic lymphangioma comprising collecting samples over time can be used in evaluating a treatment effect of measures for treating the disease when such measures were taken for a patient who is a subject between the previous sampling and the sampling this time.

The measures include administration of an agent that causes suppression of expression of amphiregulin, suppression of secretion of amphiregulin, and/or inhibition of binding of amphiregulin with an amphiregulin receptor, or an agent that causes suppression of expression of an amphiregulin receptor, suppression of activation of an amphiregulin receptor, and/or inhibition of binding of an amphiregulin receptor with amphiregulin to a patient (or a subject), administration of a pharmaceutical composition of the first aspect to a patient (or a subject), administration of a pharmaceutical composition of the second aspect to a patient (or a subject) or administration of a medicament of the fourth aspect to a patient (or a subject), administration of a known drug for cystic lymphangioma, a diet therapy, and an exercise therapy.

When the decrease in the level of amphiregulin or an amphiregulin receptor in a sample is observed when the sample before the measures and the sample after the measures are compared, it indicates that the measures are effective and can be so determined. On the other hand, when the change in the level of amphiregulin or an amphiregulin receptor in a sample is not observed or the level is increased when the sample before the measures and the sample after the measures are compared, it indicates that the measures are not effective and can be so determined.

Accordingly, the test method according to the sixth aspect of the invention may be used for screening a substance, particularly a drug, that is effective for the treatment of cystic lymphangioma. For example, a method for screening a substance that is effective for the treatment of cystic lymphangioma according to one embodiment of the invention comprises preparing lymphatic endothelial cells or fibroblast cells around one or more lymph vessels of an animal (preferably a human or mouse, more preferably a human), contacting a test substance with the cells, and detecting the change in the level of amphiregulin or an amphiregulin receptor in the cells after contacting with the test substance. The details of amphiregulin and an amphiregulin receptor are described with respect to the second aspect. The level of amphiregulin and level of an amphiregulin receptor may be the level of the protein or the level of mRNA. When the level of amphiregulin in the cells is lower after contact compared with the level before contact, it indicates that the test substance is a candidate that is effective for the treatment of cystic lymphangioma and the test substance can be selected. Alternatively, when the level of amphiregulin is the same or higher after contact compared with the level before contact, it indicates that the test substance is not a candidate that is effective for the treatment of cystic lymphangioma and can be so determined.

According to a seventh aspect of the invention, provided is a test kit for cystic lymphangioma comprising either (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.

The substance that binds DNA encoding amphiregulin of (a) includes, for example, a polynucleotide (DNA or RNA) that selectively hybridizes with DNA encoding amphiregulin. Such a polynucleotide may be easily produced or obtained by those skilled in the art by producing a polynucleotide (the length being about 15 to 25 nucleotides, for example) that has a sequence complementary to at least a portion of the DNA encoding amphiregulin. The substance that binds mRNA transcribed based on DNA encoding amphiregulin as a template includes, for example, a polynucleotide (DNA or RNA) that selectively hybridizes with mRNA transcribed based on DNA encoding amphiregulin. Such a polynucleotide may be easily produced or obtained by those skilled in the art by producing a polynucleotide (the length being about 15 to 25 nucleotides, for example) that has a sequence complementary to at least a portion of the mRNA transcribed based on DNA encoding amphiregulin.

The substance that binds DNA encoding an amphiregulin receptor of (b) includes, for example, a polypeptide (DNA or RNA) that selectively hybridizes with DNA encoding an amphiregulin receptor. Such a polynucleotide may be easily produced or obtained by those skilled in the art by producing a polynucleotide (the length being about 15 to 25 nucleotides, for example) that has a sequence complementary to at least a portion of the DNA encoding an amphiregulin receptor. The substance that binds mRNA transcribed based on DNA encoding an amphiregulin receptor as a template includes, for example, a polynucleotide (DNA or RNA) that selectively hybridizes with mRNA transcribed based on DNA encoding an amphiregulin receptor. Such a polynucleotide may be easily produced or obtained by those skilled in the art by producing a polynucleotide (the length being about 15 to 25 nucleotides, for example) that has a sequence complementary to at least a portion of the mRNA transcribed based on DNA encoding an amphiregulin receptor.

The kit may further comprise instructions for conducting a test. The kit may further comprise a container for containing either substance (a) or (b). The kit may further comprise a solvent for dissolving or suspending either substance (a) or (b). The kit may further comprise a preservative or a pH control agent.

The test using the test kit is conducted using a sample collected from a subject. The subject, the sample collected from the subject, and the test method are as described with respect to the test method according to the sixth aspect of the invention.

According to an eighth aspect of the invention, provided is a test kit for cystic lymphangioma comprising either substance (a) or (b):
(a) a pair of oligonucleotide primers for amplifying at least a portion of the nucleic acid sequence of a DNA encoding amphiregulin, an mRNA transcribed based on the DNA as a template, or a cDNA transcribed based on the mRNA; and
(b) a pair of oligonucleotide primers for amplifying at least a portion of the nucleic acid sequence of a DNA encoding an amphiregulin receptor, an mRNA transcribed based on the DNA as a template, or a cDNA transcribed based on the mRNA.

The pair of primers of (a) are designed to have sequences that are complementary with a target nucleic acid sequence of a DNA encoding amphiregulin, an mRNA transcribed based on the DNA as a template, or a cDNA transcribed based on the mRNA, and to couple with the target nucleic acid sequence at both ends of the target nucleic acid sequence. The pair of primers of (b) are designed to have sequences that are complementary with a target nucleic acid sequence of a DNA encoding an amphiregulin receptor, an mRNA transcribed based on the DNA as a template, or a cDNA transcribed based on the mRNA, and to couple with the target nucleic acid sequence at both ends of the target nucleic acid sequence. The primers preferably amplify the sequence having 10 to 200 bases. In one embodiment, the lower limit of the length of the primers is at least 12 bases, at least 15 bases, or at least 18 bases. The amplification reaction using a pair of specific primers (a forward primer and a reverse primer) is known as PCR, and design of the pair of such primers for nucleic acid amplification is well known to those skilled in the art.

The test using the test kit is conducted using a sample collected from a subject. By using the test kit for cystic lymphangioma, presence and/or amount of amphiregulin or an amphiregulin receptor in the sample may be detected. The subject, the sample collected from the subject, and the test method are as described with respect to the test method according to the sixth aspect of the invention.

The kit may further comprise instructions for conducting a test. The kit may further comprise a container for containing the first and second primers. The kit may further comprise a solvent for dissolving or suspending the first and the second primers. The kit may further comprise a preservative or a pH control agent.

The test using the test kit is conducted using a sample collected from a subject. The subject, the sample collected from the subject, and the test method are as described with respect to the test method according to the sixth aspect of the invention.

According to a ninth aspect of the invention, provided is a test agent for cystic lymphangioma comprising either substance (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.

The details of substance (a) and (b) are as described with respect to the test kit for cystic lymphangioma according to the seventh aspect.

The test is conducted using a sample collected from a subject. The subject, the sample collected from the subject, and the test method are as described with respect to the test method according to the sixth aspect of the invention.

The disclosures of all the patent applications and literature are incorporated herein by reference in their entirety.

The invention will be explained in more detail by referring to the following examples, but the invention is not limited to these examples.

### [Examples]

### Example 1: Expression of amphiregulin in fibroblast cells in lymphatic vessels in PDGFRβ knockout mice

### 1. Preparation of PDGFRβ knockout mice

PDGFRβ knockout mice were produced based on Stem Cells, 2016 Mar; 34(3): 685-98. doi: 10.1002/stem.2212; EBioMedicine, 2018 May; 31: 190-201. doi: 10.1016/j.ebiom.2018.04.021.

### 2. Immunostaining

The tissues of the PDGFRβ knockout mice, or the cells that were collected from the DGFRβ knockout mice and cultured, were fixed with paraformaldehyde (PFA). Next, the sample of the fixed tissues or cells was washed with a buffer and antigen-retrieved by heating in a microwave oven for 5 minutes using a commercial retrieval solution. Next, each sample was washed with a buffer and the tissues or the cells were subjected to a commercial blocking solution (room temperature, one hour). Next, each sample was washed with a buffer and the tissues or the cells were subjected to an anti-amphiregulin antibody (Santa Cruz Biotechnology) and an anti-LYVE1 antibody (Abcam or Thermo Fisher Scientific), both of which are primary antibodies, at optimum concentration (4°C, overnight). Next, each sample was washed with a buffer and the tissues or the cells were subjected to an antibody against the animal species that produced the anti-amphiregulin antibody (a secondary antibody having a fluorescent dye for visualization) and an antibody against the animal species that produced the anti-LYVE1 antibody (a secondary antibody having a fluorescent dye for visualization) at optimum concentration (4°C,, overnight). To dye the nucleus, Hoechst was mixed in a reaction solution of the secondary antibodies at optimum concentration. Then, each sample was washed with a buffer and mounted with a commercial mounting medium. The obtained sample was observed and photographed with a fluorescence microscope.

### 3. Western blot analysis

The tissues of PDGFRβ knockout mice and control mice, and the cells that were collected from PDGFRβ knockout mice and control mice and cultured, were lysed in a commercial lysis buffer (formation of lysates). A sample buffer containing a reduction agent was added to each lysate in an optimum amount and heated at 95°C for 5 to 10 minutes (formation of samples for western blotting). Each of the samples for western blotting was subjected to polyacrylamide electrophoresis in an optimum amount (normally, 5 to 30 µL) to separate proteins according to their molecular weight. The separated proteins in the gel were transferred to a membrane using a blotting device, and the membrane was blocked with commercial fat-free milk and washed with a buffer. Then, the membrane was subjected to an anti-amphiregulin antibody (Santa Cruz Biotechnology, a primary antibody) (4°C, overnight) and washed with a buffer. Next, the amphiregulin protein derived from the tissues or the cells on the membrane was subjected to an antibody against the animal species that produced the anti-amphiregulin antibody (Nichirei Bioscience Inc., a secondary antibody having HRP) at optimum concentration and washed with a buffer. Using a commercial chemiluminescent reagent, a band of amphiregulin was made luminous. The band of amphiregulin was photographed with a commercial imaging device.

### 4. Statistical analysis

The density of the band of amphiregulin obtained from the result of western blot analysis was measured with ImageJ imaging software. The t-test was conducted with commercial software for statistical analysis and tested for a significant difference between the PDGFRβ knockout group and the control group.

### 5. Results

As shown in Fig. 2A, according to immunostaining of fibroblasts around lymphangioma of PDGFRβ knockout mice, increase in the expression of amphiregulin was observed. As shown in Fig. 2B, when the expression of amphiregulin was compared between the control group of normal mice and the group of PDGFRβ knockout mice, a significant difference was observed in western blot analysis of the tissues.

### Example 2: Expression of amphiregulin in human cystic lymphangioma

### 1. Preparation of samples

Paraffin sections were prepared from paraffin blocks of human tissues (three specimens from patients with cystic lymphangioma and three specimens from subjects with other diseases of a control group). Prior informed consent was obtained from each subject.

### 2. Immunostaining

Paraffin sections were prepared from paraffin blocks of human tissues (three specimens from patients with cystic lymphangioma and three specimens from subjects with other diseases of a control group). Deparaffinization was conducted. The sample of the tissue sections was antigen-retrieved by heating in a microwave oven for 5 minutes using a commercial retrieval solution. Next, each sample was washed with a buffer and the tissues or the cells were subjected to a commercial blocking solution (room temperature, one hour). Next, each sample was washed with a buffer and the tissues or the cells were subjected to an anti-amphiregulin antibody (Santa Cruz Biotechnology) and an anti-podoplanin antibody (Nichirei Bioscience Inc.), both of which are primary antibodies, at optimum concentration (4°C, overnight). Next, each sample was washed with a buffer and the tissues or the cells were subjected to an antibody against the animal species that produced the anti-amphiregulin antibody (a secondary antibody having a fluorescent dye for visualization) and an antibody against the animal species that produced the anti-podoplanin antibody (a secondary antibody having a fluorescent dye for visualization) at optimum concentration (4°C, overnight). To dye the nucleus, Hoechst was mixed in a reaction solution of the secondary antibodies at optimum concentration. Then, each sample was washed with a buffer and mounted with a commercial mounting medium. The obtained sample was observed and photographed with a fluorescence microscope.

### 3. Pixel analysis

The portions that were amphiregulin-positive and podoplanin-negative (an interstitium where the cells are present) were cut randomly in the range of 50 µm × 50 µm. The number of amphiregulin-positive pixels was measured with ImageJ imaging software. 16 areas were measured for each of three specimens from patients with cystic lymphangioma and three specimens from subjects with other diseases of a control group.

### 4. Statistical processing

The t-test was conducted with commercial software for statistical analysis and tested for a significant difference between the group of patients with cystic lymphangioma and the control group.

### 5. Results

As shown in Fig. 3A, when the expression of amphiregulin was examined using samples of human cystic lymphangioma, significantly higher expression of amphiregulin was confirmed in fibroblasts around lymphangioma (lower side of Fig. 3A) compared with the control group (upper side of Fig. 3A).

The endothelial cells in the cystic lymph vessels also expressed amphiregulin (Fig. 3A) and the expression of amphiregulin in lymphatic endothelial cells significantly increased in co-culture of the fibroblasts and lymphatic endothelial cells (data not shown). Thus, it was revealed that the expression of amphiregulin in fibroblast cells induced expression of amphiregulin in lymphatic endothelial cells as a trigger, and amphiregulin of these two sources induced abnormal proliferation of lymphatic endothelial cells via amphiregulin receptors expressed in lymphatic endothelial cells.

As shown in Fig. 3B, when the expression of amphiregulin between the control group, i.e., fibroblasts of a normal human, and fibroblasts of patients with cystic lymphangioma were compared, a significant difference was observed in pixel analysis of the tissues.

### Example 3: Expression of an amphiregulin receptor in human cystic lymphangioma

### 1. Preparation of the sample

Paraffin sections were prepared from paraffin blocks of human tissues (three specimens from patients with cystic lymphangioma and three specimens from subjects with other diseases of a control group). Prior informed consent was obtained from each subject.

### 2. Immunostaining

Paraffin sections were prepared from paraffin blocks of human tissues (three specimens from patients with cystic lymphangioma and three specimens from subjects with other diseases of a control group). Deparaffinization was conducted. The sample of the tissue sections was antigen-retrieved by heating in a microwave oven for 5 minutes using a commercial retrieval solution. Next, each sample was washed with a buffer and the tissues or the cells were subjected to a commercial blocking solution (room temperature, one hour).

Next, each sample was washed with a buffer and the tissues or the cells were subjected to an anti-phosphorylated EGFR antibody (Cell Signaling Technology) and an anti-podoplanin antibody (Nichirei Bioscience Inc.), both of which are primary antibodies, at optimum concentration (4°C, overnight). Next, each sample was washed with a buffer and the tissues or the cells were subjected to an antibody against the animal species that produced the anti-phosphorylated EGFR antibody (Thermo Fisher Scientific) (a secondary antibody having a fluorescent dye for visualization) and an antibody against the animal species that produced the anti-podoplanin antibody (Thermo Fisher Scientific) (a secondary antibody having a fluorescent dye for visualization) at optimum concentration (4°C, overnight). To dye the nucleus, Hoechst was mixed in a reaction solution of the secondary antibodies at optimum concentration. Then, each sample was washed with a buffer and mounted with a commercial mounting medium. The obtained sample was observed and photographed with a fluorescence microscope.

### 3. Results

As shown Fig. 4, a signal of an amphiregulin receptor was activated in cystic lymphatic endothelial cells in tissues derived from patients with human cystic lymphangioma (image of phosphorylated EGFR extremely positive; lower side of Fig. 4), which supports abnormal proliferation of lymphatic endothelial cells. In the control group (upper side of Fig. 4), the signal of an amphiregulin receptor was at an extremely lower level and phosphorylated EGFR positive level was lower than that of the control group.

### Example 4: Therapy experiment in cystic lymphangioma of PDGFRβ knockout mice

### 1. Preparation of PDGFRβ knockout mice

PDGFRβ knockout mice were produced based on Stem Cells, 2016 Mar; 34(3): 685-98. doi: 10.1002/stem.2212; EBioMedicine, 2018 May;31: 190-201. doi: 10.1016/j.ebiom.2018.04.021.

### 2. Administration of a therapeutic agent

To PDGFRβ knockout mice, a neutralizing antibody against amphiregulin (AF989, R&D Systems Inc.) and Erlotinib having EGFR inhibitory activity (Selleck Biotech) were administered for the purpose of treating cystic lymphangioma. The neutralizing antibody against amphiregulin was dissolved in PBS at 25 µg/mL and administered intraperitoneally at 5 µg/body three times a week. Erlotinib was dissolved in DMSO at 50 mg/mL and administered intraperitoneally at 50 mg/kg three times a week. A group of PDGFRβ knockout mice without treatment and a control group of mice in which PDGFRβ was not knocked out were also prepared.

### 3. Immunostaining

The tissues of PDGFRβ knockout mice were fixed with paraformaldehyde (PFA). Paraffin sections were prepared using fixed samples. The tissue sections were washed with a buffer and antigen-retrieved by heating in a microwave oven for 5 minutes using a commercial retrieval solution. Next, each sample was washed with a buffer solution and the tissues or the cells were subjected to a commercial blocking solution (room temperature, one hour).

Next, each sample was washed with a buffer and subjected to an anti-LYVE1 antibody (Abcam, a primary antibody) at optimum concentration (4°C, overnight). Next, each sample was washed with a buffer and subjected to an antibody against the animal species that produced the anti-LYVE1 antibody (Nichirei Bioscience Inc., a secondary antibody having HRP for visualization) at optimum concentration (4°C, overnight). Next, each sample was washed with a buffer and subjected to DAB to visualize lymphatic endothelial cells. To dye the nucleus, hematoxylin action was performed at optimum concentration. Then, each sample was washed with a buffer and mounted with a commercial mounting medium. The obtained sample was observed and photographed with a fluorescence microscope.

### 4. Statistical processing

The diameter of each lymph vessel was measured with ImageJ imaging software based on the photographed images. A test was conducted with commercial software for statistical analysis and tested for a significant difference between the group of PDGFRβ knockout mice with treatment, the group of PDGFRβ knockout mice without treatment, and the control group of mice in which PDGFRβ was not knocked out.

### 5. Results

As shown in Figs. 5A and 5B, the diameter of dilated lymph vessels of PDGFRβ knockout mice was significantly reduced by the administration of Erlotinib or the neutralizing antibody of amphiregulin compared with the group of PDGFRβ knockout mice without treatment. It was indicated that the diameter of the vessels of PDGFRβ knockout mice with treatment was recovered to the same extent as the control group of mice in which PDGFRβ was not knocked out.

### Industrial Applicability

The prevent invention enables development or provision of a molecular-targeted therapeutic drug against human cystic lymphangioma for which there exists no basic therapeutic agent. It is expected that the agent that causes suppression of expression of amphiregulin, suppression of secretion of amphiregulin, and/or inhibition of binding of amphiregulin with an amphiregulin receptor, and the agent that causes suppression of expression of an amphiregulin receptor, suppression of activation of an amphiregulin receptor, and/or inhibition of binding of an amphiregulin receptor with amphiregulin, will each become an effective medicament.

## Claims

1. A pharmaceutical composition for preventing or treating cystic lymphangioma comprising an agent that causes suppression of expression of amphiregulin, suppression of secretion of amphiregulin, and/or inhibition of binding of amphiregulin with an amphiregulin receptor, as an active agent.

2. The pharmaceutical composition according to claim 1, wherein the agent is selected from the group consisting of a neutralizing antibody against amphiregulin, an antigen binding fragment of a neutralizing antibody against amphiregulin, an antisense oligonucleotide against amphiregulin, a small interfering RNA against amphiregulin, a ribozyme against amphiregulin, and an aptamer against amphiregulin.

3. A pharmaceutical composition for preventing or treating cystic lymphangioma comprising an agent that causes suppression of expression of an amphiregulin receptor, suppression of activation of an amphiregulin receptor, and/or inhibition of binding of an amphiregulin receptor with amphiregulin, as an active ingredient.

4. The pharmaceutical composition according to claim 3, wherein the agent is selected from the group consisting of a neutralizing antibody against an amphiregulin receptor, an antigen binding fragment of a neutralizing antibody against an amphiregulin receptor, an antisense oligonucleotide against an amphiregulin receptor, a small interfering RNA against an amphiregulin receptor, a ribozyme against an amphiregulin receptor, an aptamer against an amphiregulin receptor, and a low-molecular compound that inhibits proliferation of lymphatic endothelial cells via an amphiregulin receptor.

5. Use of an agent of either (a) or (b) for manufacturing a medicament for preventing or treating cystic lymphangioma:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

6. A medicament for inhibiting proliferation of lymphatic endothelial cells comprising an agent of either (a) or (b) as an active ingredient:
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

7. A kit for preventing or treating cystic lymphangioma comprising an agent of either (a) or (b):
(a) an agent that causes suppression of expression of amphiregulin, suppression of secretion of amphiregulin, and/or inhibition of binding of amphiregulin with an amphiregulin receptor; and
(b) an agent that causes suppression of expression of an amphiregulin receptor, suppression of activation of an amphiregulin receptor, and/or inhibition of binding of an amphiregulin receptor with amphiregulin.

8. A method for testing cystic lymphangioma comprising measuring amphiregulin or an amphiregulin receptor in a sample collected from a subject.

9. A test kit for cystic lymphangioma comprising either (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.

10. A test agent for cystic lymphangioma comprising either substance of (a) or (b):
(a) a substance that binds amphiregulin selected from the group consisting of a neutralizing antibody against amphiregulin, an antigen binding fragment of a neutralizing antibody against amphiregulin and an aptamer against amphiregulin, a substance that binds DNA encoding amphiregulin, a substance that binds mRNA transcribed based on DNA encoding amphiregulin as a template, and cDNA reverse-transcribed based on amphiregulin mRNA as a template; and
(b) a substance that binds an amphiregulin receptor selected from the group consisting of a neutralizing antibody against an amphiregulin receptor, an antigen binding fragment of a neutralizing antibody against an amphiregulin receptor and an aptamer against an amphiregulin receptor, a substance that binds DNA encoding an amphiregulin receptor, a substance that binds mRNA transcribed based on DNA encoding an amphiregulin receptor as a template, and cDNA reverse-transcribed based on amphiregulin receptor mRNA as a template.
